# EUROPEAN PATENT SPECIFICATION

(11) **EP 0 932 613 B1**
(45) Date of publication and mention of the grant of the patent: **09.03.2005**
(21) Application number: 97927727.4
(22) Date of filing: 22.05.1997
(51) Int. Cl.: C07K 7/06, A61K 38/08

(54) **BLOCKING EXPRESSION OF VIRULENCE FACTORS IN S. AUREUS**
EXPRESSIONSBLOCKIERUNG VON VIRULENTEN FAKTOREN IN S. AUREUS
BLOCAGE DE L'EXPRESSION DE L'AGGRESSINE DANS S. AUREUS

(30) Priority: 22.05.1996 US 651226
(43) Date of publication of application: 04.08.1999
(73) Proprietor: NEW YORK UNIVERSITY, New York, NY 10016 (US)
(72) Inventor: NOVICK, Richard, P., New York, NY 10024 (US); JI, Guangyong, Elmhurst, NY 11373 (US); BEAVIS, Ronald, Indianapolis, IN 46234 (US)
(74) Representative: COHAUSZ & FLORACK
(86) International application number: PCT/US1997/008791
(87) International publication number: WO 1997/044349

(56) References cited:
- WO-A-96/10579
- JI G ET AL: "Cell density control of staphylococcal virulence mediated by an octapeptide pheromone" PROCEEDINGS OF THE NATIONAL ACADEMY OF SCIENCES OF USA,US,NATIONAL ACADEMY OF SCIENCE. WASHINGTON, vol. 92, 1 December 1995 (1995-12-01), pages 12055-12059, XP002081593 ISSN: 0027-8424
- DATABASE MEDLINE [Online] US NATIONAL LIBRARY OF MEDICINE (NLM), BETHESDA, MD, US; VANDENESCH F ET AL: "Agr-related sequences in Staphylococcus lugdunensis." retrieved from STN Database accession no. 93366158 XP002181146 & FEMS MICROBIOLOGY LETTERS, (1993 JUL 15) 111 (1) 115-22. ,
- MOLECULAR GENETICS, 1995, Volume 248, NOVICK R.P. et al., "The agr P2 Operon: an Autocatalytic Sensory Transduction System in Staphylococcus Aureus", pages 446-458, XP002030540.
- PROC. NATL. ACAD. SCI. U.S.A., December 1995, Volume 92, No. 6, JI et al., "Cell Density Control of Staphylococcal Virulence Mediated by an Octapeptide Pheromone", pages 12055-12059, XP002081593.

## Description

This invention was made under NIH Grant No. Al-R01-30138. As such, the Government has certain rights in the invention.

### BACKGROUND OF THE INVENTION

Staphylococcus aureus (S. aureus) is Gram-positive, aerobic bacterial pathogen, distinguished from other staphylococcal species by the production of the enzyme coagulase. Sa is a normal inhabitant of the skin and mucous membranes of man and other animals and under certain circumstances invades the body, causing a wide variety of disease conditions ranging from superficial abscesses (boils and furuncles) to disfiguring and life-threatening deep infections such as endocarditis, pneumonia, osteomyelitis, septic arthritis, meningitis, post-operative wound infections, and septicemia. Sa also causes diseases such as toxic shock syndrome.

Like other Gram-positive pathogens, Sa causes disease chiefly through the production and secretion of injurious proteins. These injurious extracellular proteins, or virulence factors (VF), include toxins that damage or dissolve host cells, toxins that interfere with the immune system, and enzymes that degrade tissue components such as proteins, nucleic acids, lipids and polysaccharides.

In laboratory cultures, VF are produced and secreted at the end of the standard exponential growth phase, during a segment of the growth cycle known as the post-exponential phase. The production of VF is coordinately regulated and is thought to represent an attempt by the bacteria to generate new sources of nutrition at a time of rapidly diminishing resources. In the infected individual, this may include an attack on the host defenses that have been mobilized to ward off and contain the infection.

Sa infections are presently treated with antibiotics, which are natural or semisynthetic chemicals that kill or inhibit the growth of bacterial cells. Unfortunately, antibiotics have become less and less effective in treating Sa infections due to the acquired resistance of Sa to these antibiotics. Major nosocomial epidemics are now caused worldwide by strains of Sa that are resistant to most antibiotics. The antibiotic vancomycin is still effective in treating various strains of Sa, although there is a grave danger that those strains will soon acquire resistance to vancomycin from a closely related Gram-positive pathogen, Enterococcus faecalis.

Since there is little reason to expect the introduction of major new classes of antibiotics, there is an urgent need to develop new methods to control Sa infections, such as interference with the expression of VF. If the bacteria could be disarmed, it is believed that host defenses would do the rest.

In S. aureus, expression of virulence factors is controlled by a global regulator known as agr (Peng, H., et al. J. Bacteriol. 179: 4365-4372 (1988); Regassa, L.B., et al. Infect. Immun. 60: 3381-3383 (1992)). Agr is a genetic locus that contains several genes. Two of these, agrA and agrC, are thought to constitute a signal transduction (STR) pathway that responds to one or more external signalling molecules by activating the transcription of a third gene, agr-rnaIII (Kornblum, J., et al., in Molecular Biology of the Staphylococci, R.P. Novick, ed. (VCH Publishers, New York, 1990); Bourret, R.B., et al. Annu. Rev. Biochem. 60: 401-441 (1991)). The primary transcript of agr-rnaIII, known as RNAIII, induces transcription of the 20 or more independent genes encoding virulence factors, thereby resulting in the synthesis of VF (Novick, R.P., et al. EMBO Journal 12(10): 3967-3975 (1993)).

It has been shown that laboratory-generated mutant strains of Sa, unable to express VF, exhibit greatly reduced virulence (Foster, et al. Molecular Biology of the Staphylococci, Editor: R.P. Novick, VCH Publisher, New York, pp. 403-420 (1990)). Interference with activation of the agr system would therefore afford a simple means of blocking the expression of VF, and thus interfere wiht the infective process. Raychoudhury, S. et al. PNAS 90:965-969 (1993) recently described the identification of synthetic chemical compounds that block the expression of alginate, a VF for the cystic fibrosis pathogen, Pseudomonas aeruginosa. It has not been shown, however, whether these chemicals would have any effect on Sa, of offer any potential clinical utility.
PNAS 92:12055-12059 (1995) discloses the peptide YSTCDFIM to activate expression of the *agr* locus, a global regulator of the virulence response.

### SUMMARY OF THE INVENTION

The present invention is based upon the discovery of peptides which interfere with the activation of rnaIII transcription and thus prevent expression of VF. The invention is directed to a medicament comprising a purified peptide which inhibits a agr-rnaIII transcription in S. aureus, the peptide containing six to twelve amino acids in length and an analogue thereof which inhibits agr-rnaIII transcription in S. aureus, and a pharmaceutically acceptable carrier. Further subject matters are the use of the aforementioned peptide in the manufacture of a medicament for the treatment of prevention of an infection or disease caused by S. aureus as well as a non-therapeutical method of inhibiting agr-rnaIII transcription in S. aureus.

### BRIEF DESCRIPTION OF THE FIGURES

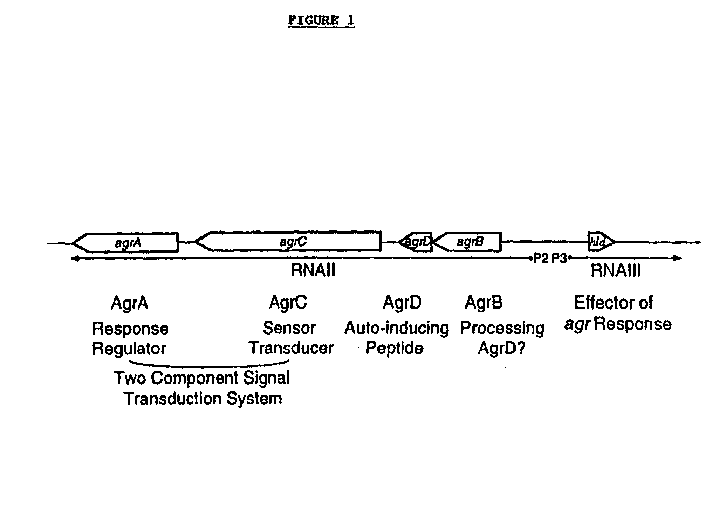

Figure 1 represents the agr locus of S. aureus. Schematic map of the agr locus showing the major transcripts RNAII and RNAIII (arrows) and the genes indicated by boxes.

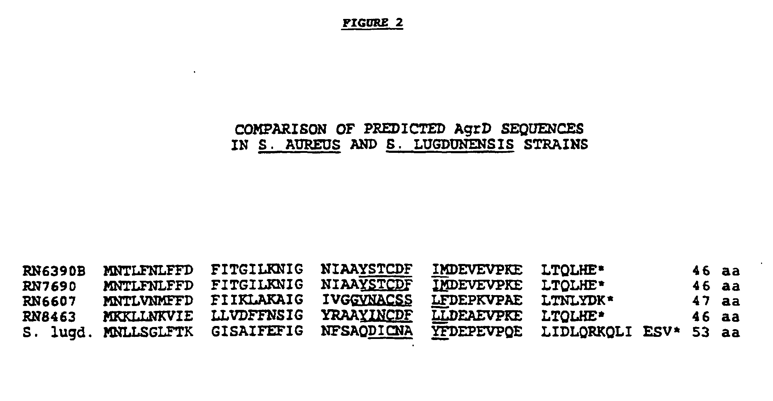

Figure 2 represents a comparison of the predicted AgrD sequences of S. aureus strains RN6390B (laboratory strain), RN 7690 (clinical isolate), RN 6607 (clinical isolate), RN8463 (clinical isolate) and S. lugdunesnis. The underlined region corresponds to the identification of the activator peptide from RN6309B and the inhibitor peptide from S. lugdunensis.

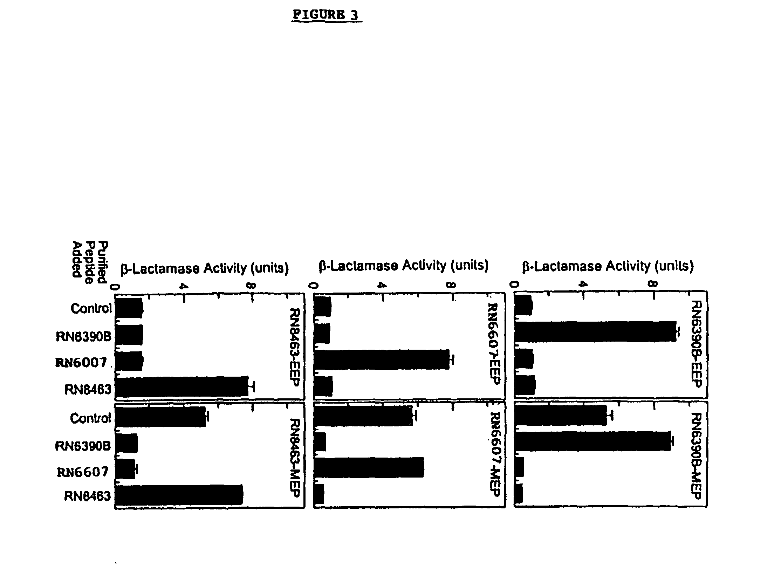

Figure 3 represents the effects of purified peptide from S. aureus strains RN6390B, RN6607 and RN8463 on the RNAIII transcription of early exponential phase (EEP) and mid-exponential phase (MEP) cultured S. aureus strains RN6390B, RN6607 and RN8463. The RNAIII transcription was measured as described in Ji, G., et al. PNAS USA 92:12055-12059 (1995) with 10 mM Tris-HCl, pH 7.5 as the control.

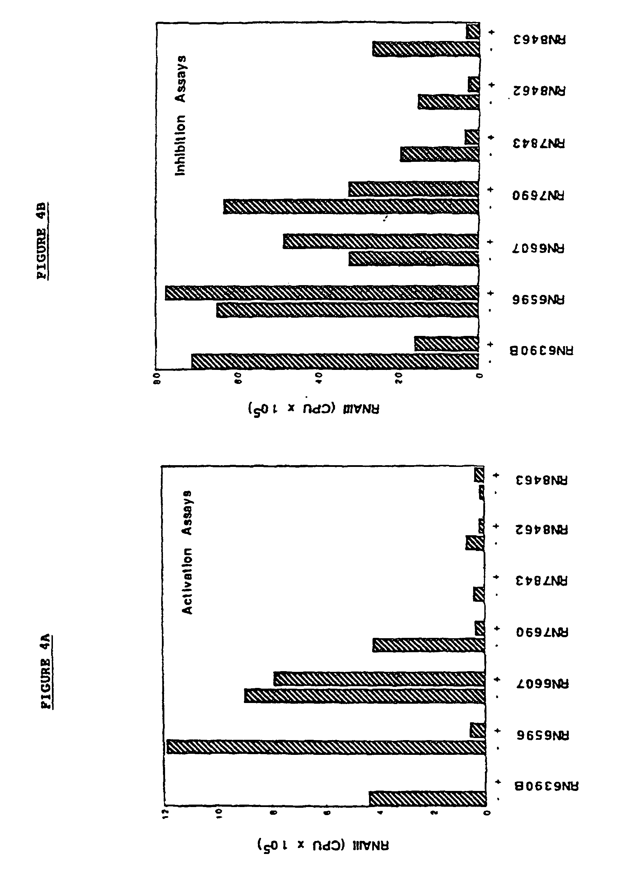

Figures 4A and 4B represent the effect of S. lugdunensis pheromone on the RNAIII transcription of various S. aureus strains: RN6390B (laboratory strain), RN6596 (laboratory strain), RN6607 (clinical isolate), RN7690 (clinical isolate), RN7843 (clinical isolate), RN8462 (TSST) and RN8463 (TSST). Figure 4A, activation assays; Figure 4B, inhibition assays.

### DETAILED DESCRIPTION OF THE INVENTION

The present invention provides purified and isolated peptides which inhibit agr-rnaIII transcription in S. aureus. The peptides of the present invention are cyclic, comprise about six to about twelve amino acids in length, and include amino acid number 28 from the AgrD region of a staphylococci bacterium. As used herein, amino acid number 28 of the agrD region of a staphylococci bacterium corresponds to the "cysteine" shown in Figure 2, which is believed to be conserved in the corresponding AgrD regions of various staphylococci bacterium. The staphylococci bacterium includes but is not limited to S. aureus, S. capitis, S. caprae, S. carnosus, S. caseolyticus, S. epidermidis, S. haemolyticus, S. hominis, S. hyicus subsp. hyicus, S. hyicus subsp. chromo, S. kloosii, S. lentus, S. lugdunensis, S. sciuri, S. simulans and S . xylosus.

The inhibitor peptides of the present invention include sequences corresponding to the native peptide from staphylococci bacterium, as well as analogues thereof which contain amino acid substitutions which also result in the peptides being able to inhibit agr-rnaIII transcription in S. aureus. The purified inhibitor peptides of the present invention may be isolated directly from staphylococci bacterium, recombinantly produced, or synthesized chemically using procedures known in the art. Preferably, the peptides are synthesized chemically. Specific examples of the inhibitor peptides include but are not limited to the following amino acid sequences: NH₂-Asp-Ile-Cys-Asn-Ala-Tyr-Phe-COOH,NH₂-Tyr-Ser-Thr-Cys-Asp-Phe-Ile-Met-COOH,NH₂-Gly-Val-Asn-Ala-Cys-Ser-Ser-Leu-Phe-COOH and NH₂-Tyr-Ile-Asn-Cys-Asp-Phe-Leu-Leu-COOH, wherein each peptide contains a cyclic thioester bond between the cysteine and the COOH end. The synthesis of peptides containing cyclic thioester bonds between the cysteine and the COOH end is within the purview of one skilled in the art. It is also within the confines of the present invention that the cyclic bond can be a bond other than a thioester bond, such as a disulfide bond, for example, which can be obtained by adding a cysteine residue at the carboxyl end, so long that such a modification results in a peptide having inhibitor activity.

The present invention also provides a peptide composition comprising one or more of the inhibitor peptides and a pharmaceutically or physiologically acceptable carrier. The carrier must be "acceptable" in the sense of being compatible with the other ingredients of the formulation and not deleterious to the recipient thereof. Examples of suitable pharmaceutical carriers include lactose, sucrose, starch, talc, magnesium stearate, crystalline cellulose, methyl cellulose, carboxymethyl cellulose, glycerin, sodium alginate, gum arabic, powders, saline, water, among others. The choice of carrier will depend upon the route of administration. The formulations may conveniently be presented in unit dosage and may be prepared by methods well-known in the pharmaceutical art, by bringing the peptide(s) into association with a carrier or diluent, as a suspension or solution, and optionally one or more accessory ingredients, e.g. buffers, surface active agents, and the like.

The present invention also provides a method for treating or preventing an infection or disease caused by S. aureus in a subject comprising administering to the subject one or more of the inhibitor peptides in an amount effective to treat or prevent the infection or disease caused by S. aureus. The subject may be human or animal and is preferably is human. The peptide(s) alone, or conjunction with a suitable pharmaceutically acceptable carrier, may be administered by procedures known to those skilled in the art, including but not limited to parenteral (i.e., intravenous, intramuscular, subcutaneous, or intraperitoneal administration), oral, sublingual and topical administration. The actual dose of the peptide(s) administered will depend upon the route of administration, the pharmacokinetics properties of the individual treated, as well as the results desired, and is readily determinable by one skilled in the art. It is also within the confines of the present invention that the peptide(s) may be administered in combination with traditional antibiotics which are used to treat diseases or infections caused by S. aureus.

The present invention is described in the following Examples which are set forth to aid in an understanding of the invention, and should not be construed to limit in any way the invention as defined in the claims which follow thereafter.

### Example 1

### Effect of Conditioned-Media of Staphylococci on the Agr Expression of S. aureus RN6390B

Various culture supernatants from 172 S. aureus and 15 other staphylococci were grown in CYGP broth at 37°C for 6 hours starting at a cell density of 2 x 10⁹ cells/ml. Cells were removed by centrifugation at 4°C. The supernatant was boiled for 10 minutes, filtered (0.22 µm filter), centrifuged, filtered with a Centricon 3 filter (3 kDa cutoff). The supernatant from these S. aureus strains and other staphylococci were then analyzed using the procedures described in Ji, G., et al. PNAS USA 92:12055-12059 (1995) for their ability to activate or inhibit agr transcription of S. aureus RN6309B. The results are presented in Tables 1 and 2. These strains can be divided into four groups as follows. In groups I, II and III (all S. aureus), the members of any one group produce a substance that activates the virulence response (agr transcription) in any other member of the same group but inhibits the response in any member of either of the other two groups. It is believed that this substance may have activation or inhibition properties depending on the strain being tested. In group IV (several staphylococcal species other than S. aureus), each of the members produces a substance that inhibits the response in RN6390B, the standard strain from group I. The substances produced by group IV strains have little or no agr-activating activity with any member of the group.

### Example 2

### Purification of RN6390B S. Aureus Peptide Using C18 Reverse Phase HPLC

S. aureus strain RN 7668 (pRN6911) was grown in tryptophan assay medium plus 50 µg/ml of L-tryptophan and 5 µg/ml CBAP starting at 2 x 10⁹ cells/ml. Before use, the medium was dialyzed with a 2 kDA cutoff membrane, discarding the contents of the membrane sac. After 6 hours of growth at 37°C, cells were removed by centrifugation and the culture supernatant was filtered (0.22 µm filter), boiled for 10 minutes, lyophilized and resuspended in 2.5% acetonitrile/ 0.1% trifluoroacetic acid (1/40 volume of the culture supernatant). This material (3 ml) was loaded onto an HPLC C18 column in 2.5% acetonitrile/ 0.1% trifluoroacetic acid, and eluted with an acetonitrile gradient (16-48%) at 0.27% acetonitrile per minute. The collected fractions (1.5 ml per fraction) were lyophilized and suspended in 0.1 ml of 20 mM Tris-HCL buffer (pH 7.5). Fractions with activator pheromone activity were pooled and filtered through a Centricon 3 filter with 3 kDa cutoff. The filtrate (1 ml) was rerun on the HPLC C-18 column and eluted with an acetonitrile gradient at 0.2% acetonitrile per minute over the interest range (20-30%).

The activator peptide, eluting at an acetonitrile concentration of about 28.5%, was analyzed by matrix-assisted laser desorption/ionization mass spectrometry (MALDI-MS) (Hillenkamp, F., et al. Anal. Chem. 63:1193A-1203A (1991)) and its amino acid sequence was determined to be Tyr-Ser-Thr-Cys-Asp-Phe-Ile-Met by a Procise Edman Sequencer (Perkin-Elmer), which is located within the AgrD gene as shown in Figure 2. MALDI-MS was performed using a linear time-of-flight mass spectrometer with a nitrogen laser ion source custom-built at New York University. The matrix used was α-cyano-4-hydroxycinnamic acid and the sample was prepared using the droplet method.

The purified activator peptide was then analyzed for its ability activate and inhibit RNAIII transcription of S. aureus strains RN6390B, RN6607 and RN8463, as described in Ji, G., et al. PNAS USA 92:12055-12059 (1995). The results are presented in Figure 3, which show that the activator peptide had an activator effect on strain RN6390B, and an inhibitor effect on strains RN6607 and RN8463.

### Example 3

### Purification of Other S. Aureus Peptides Using C18 Reverse Phase HPLC

S. aureus strain RN 7667 containing cloned agrBD genes of either RN6607 or RN8463 was grown in tryptophan assay medium plus 50 µg/ml of L-tryptophan and 5 µg/ml CBAP starting at 2 x 10⁹ cells/ml. After 6 hours of growth, cells were removed by centrifugation and the culture supernatant was filtered (0.22 µm filter), boiled for 10 minutes, lyophilized and suspended in solution A (2.5% acetonitrile plus 0.1% trifluoroacetic acid). This material was loaded onto a Sephasil C18 (Pharmacia) column (3 cm x 5 cm), washed once with solution A, once with solution B (15% acetonitrile plus 0.1% trifluoroacetic acid) and eluted with solution C (40% acetonitrile plus 0.1% trifluoroacetic acid). The eluted material was lyophilized, suspended in 20 mM Tris-HCL buffer (pH 7.5) and filtered through a Centricon 3 filter (Amicon). The filtrate was then loaded onto an HPLC C-18 column and eluted with an acetonitrile gradient (16-32%) at 0.2% acetonitrile per minute. Fractions with activity were pooled, lyophilized, suspended in 20 mM Tris-HCL buffer (pH 7.5) and analyzed by MALDI-MS, and its amino acid sequence was determined by a Perkin-Elmer Procise Edman Sequencer. The amino acid sequences for RN6607 and RN8463 were determined to be Gly-Val-Asn-Ala-Cys-Ser-Ser-Leu-Phe and Tyr-Ile-Asn-Cys-Asp-Phe-Leu-Leu, respectively, which are located in the same region of agrD as the activator peptide from S. aureus strain RN6390B as shown in Figure 2.

The purified peptide were then analyzed for their ability activate and inhibit RNAIII transcription of S. aureus strains RN6390B, RN6607 and RN8463, as described in Ji, G., et al. PNAS USA 92:12055-12059 (1995). The peptide from RN6607 had an activator effect on strain RN6607, and an inhibitor effect on strains RN6390B and RN8463, while the peptide from RN8463 had an activator effect on strain RN8463, and an inhibitor effect on strains RN6390B and RN6607, as shown in Figure 3.

### Example 4

### Purification and Analysis of Inhibitor Peptide From S. lugdunensis

S. aureus strain RN 7668 (pSLBD) was grown in methionine assay medium plus 50 µg/ml of L-methionine and 5 µg/ml CBAP starting at 2 x 10⁹ cells/ml. Before use, the medium was dialyzed with a 2 kDA cutoff membrane, discarding the contents of the membrane sac. After 6 hours of growth at 37°C, cells were removed by centrifugation and the culture supernatant was boiled for 10 minutes and filtered (0.22 µm filter). This material was loaded onto a Sephasil C18 column (3.5 x 4 cm), washed with (i) 2.5% acetonitrile, 0.1% trifluoroacetic acid, (ii) 10.5% acetonitrile, 0.1% trifluoroacetic acid. The inhibitor was eluted with 42.5% acetonitrile, 0.1% trifluoroacetic acid, lyophilized, suspended in 20 mM Tris-HCl buffer (pH 7.5) and filtered through a Centricon 3 filter with a 3 kDa cutoff. This material was then loaded onto an HPLC C-18 column and eluted with an acetonitrile gradient. Fractions with inhibitor pheromone activity were pooled, lyophilized, dissolved in 20 mM Tris-HCL buffer (pH 7.5) and stored at -80°C. The fractions were analyzed by MALDI-MS, and using Edman degradation procedure, this peptide was determined to have the amino sequence Asp-Ile-Cys-Asn-Ala-Tyr-Phe, which is located in the same region of agrD as the activator peptide from S. aureus strain RN6390B as shown in Figure 2.

The purified S. lugdunensis pheromone was then analyzed for its ability activate and inhibit RNAIII transcription of various S. aureus strains. S. aureus strains were grown to 30 Klett units (for activation assays) and to 60 Klett units (for inhibition assays). The purified S . lugdunensis pheromone was added and the mixtures were incubated at 37°C for 30 minutes. Whole cell lysates were prepared from these cultures and analyzed by Northern blot hybridization using a RN6390B RNAIII-specific probe. The S. lugdunensis pheromone inhibits the agr response in 5 of 6 S. aureus strains tested as shown in Figures 4A and 4B.

### Example 5

### Commercial Synthesis of Synthetic Peptide Corresponding to RN6390B and S. lugdunensis Sequences

Peptides having the same amino acid sequences as the native peptides from RN6390B and S. lugdunensis were synthesized commercially (Yale University, New Haven, CT) and analyzed by MALDI-MS. Unlike the purified peptides, the synthesized peptides did not have activity. Mass spectroscopy showed that the synthetic peptides were dimeric, whereas the native peptide molecules were monomeric and had molecular masses that were 18±1 atomic mass units less than those predicted by their respective amino acid sequences. Taken together, these results suggest that the cysteines in the synthetic peptides had spontaneously formed intermolecular disulfides, whereas those in the native peptides were involved in an intramolecular bond, most likely a cyclic thioester introduced post-translationally and involving the C-terminal carboxyl, since there is no other conserved carboxyl group in the molecule. Consistent with this possibility were the results of treatment of the native peptides with iodoacetic acid and hydroxylamine. Iodoacetic acid, expected to react with free -SH groups, had no effect, whereas hydroxylamine, expected to react with thioesters, abolished activity.

### Example 6

### Synthesis of a Synthetic Peptide Corresponding to the RN8463 Sequence

We have succeeded in synthesizing a small quantity of a cyclic thioester derivative of the RN8463 octapeptide and have shown that the synthetic material inhibits agr expression by RN6390B. It is expected that the introduction of the cyclic thioester bond to the other peptide will have a similar effect on activity.

**Table 1.**

| Effect of Conditioned-Media of Staphylococci on the Agr Expression of *S. aureus* RN6390B | | |
|---|---|---|
| Strains | Activation | Inhibition |
| *S. aureus* | | |
| Animal Mastitis Isolates (6) | 4 | 2 |
| Capsule Strains (3) | 0 | 3 |
| Clinical Isolates (39) | 17 | 22 |
| Coag⁻ (9) | 0 | 9 |
| Laboratory Strains (4) | 1 | 3 |
| MRSA (65) | 13 | 52 |
| TSST-1⁺ (46) | 3 | 43 |
| *S. capitis, S. caprae, S. camosus,* | | |
| *S. caseolyticus, S epidermidis,* | | |
| *S. haemolyticus, S hominis,* | | |
| *S. hyicus* subsp. hyicus, | | |
| *S. hyicus* subsp. chromo, | 0 | All |
| *S. kloosii, S. lentus,* | | |
| *S. lugdunensis, S. sciuri,* | | |
| *S. simulans, S. xylosus* | | |

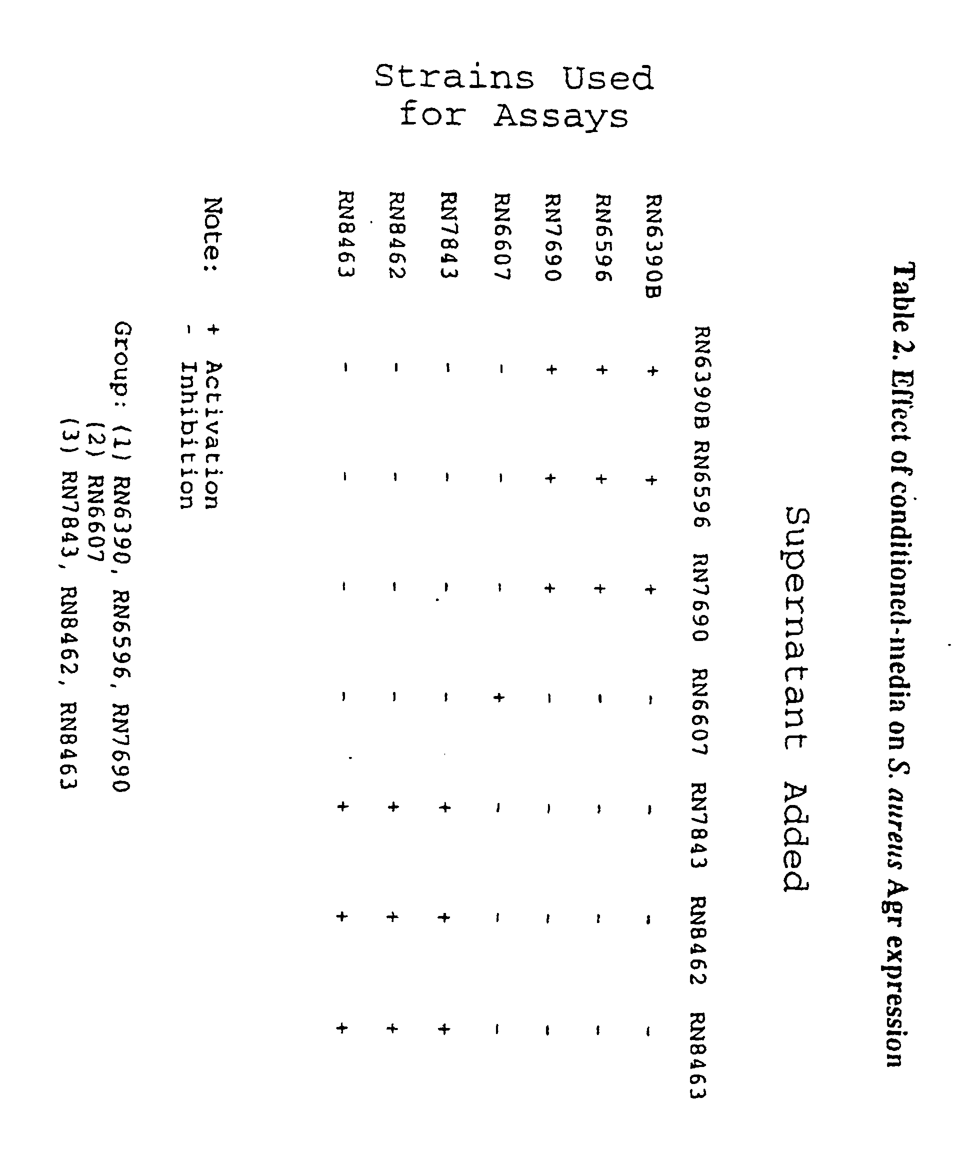

## Claims

1. A medicament comprising a purified peptide which inhibits agr-rnaIII transcription in S. aureus, the peptide containing six to twelve amino acids in length and comprising amino acid number 28 from the AgrD region of a staphylococci bacterium, or an analogue thereof which inhibits agr-rnaIII transcription in S. aureus, and a pharmaceutically acceptable carrier.

2. The medicament of claim 1, wherein the staphylococci bacterium is selected from the group consisting of S. aureus, S. capitis, S. caprae, S. carnosus, S. caseolyticus, S. epidermidis, S. haemolyticus, S. hominis, S. hyicus subsp. hyicus, S. hyicus subsp. chromo, S. kloosii, S. lentus, S. lugdunensis, S. sciuri, S. simulans and S. xylosus.

3. The medicament of claim 2, wherein the staphylococci bacterium is S. lugdunensis.

4. The medicament of claim 3, the peptide having the amino acid sequence NH₂-Asp-Ile-Cys-Asn-Ala-Tyr-Phe-COOH and a cyclic thioester bond between Cys and COOH.

5. The medicament of claim 2, wherein the staphylococci bacterium is S. aureus.

6. The medicament of claim 5, the peptide having the amino acid sequence NH₂-Tyr-Ser-Thr-Cys-Asp-Phe-Ile-Met-COOH and a cyclic thioester bond between Cys and COOH.

7. The medicament of claim 5, the peptide having the amino acid sequence NH₂-Gly-Val-Asn-Ala-Cys-Ser-Ser-Leu-Phe-COOH and a cyclic thioester bond between Cys and COOH.

8. The medicament of claim 5, the peptide having the amino acid sequence NH₂-Tyr-Ile-Asn-Cys-Asp-Phe-Leu-Leu-COOH and a cyclic thioester bond between Cys and COOH.

9. The medicament of claim 1, comprising more than one peptide which inhibits agr-rnaIII transcription in S. aureus.

10. Use of a peptide which inhibits agr-rnaIII transcription in S. aureus for the manufacture of a medicament for the treatment or prevention of an infection or disease caused by S. aureus, wherein the peptide contains six to twelve amino acids in length and comprises amino acid number 28 from the AgrD region of a staphylococci bacterium, or an analogue thereof which inhibits agr-rnaIII transcription in S. aureus.

11. The use of claim 10, wherein the staphylococci bacterium is selected from the group consisting of S. aureus, S. capitis, S. caprae, S. carnosus, S. caseolyticus, S. epidermidis, S. haemolyticus, S. hominis, S. hyicus subsp. hyicus, S. hyicus subsp. chromo, S. kloosii, S. lentus, S. lugdunensis, S. sciuri, S. simulans and S. xylosus.

12. The use of claim 11, wherein the staphylococci bacterium is S. lugdunensis.

13. The use of claim 12, the peptide having the amino acid sequence NH₂-Asp-Ile-Cys-Asn-Ala-Tyr-Phe-COOH and a cyclic thioester bond between Cys and COOH.

14. The use of claim 11, wherein the staphylococci bacterium is S. aureus.

15. The use of claim 14, the peptide having the amino acid sequence NH₂-Tyr-Ser-Thr-Cys-Asp-Phe-Ile-Met-COOH and a cyclic thioester bond between Cys and COOH.

16. The use of claim 14, the peptide having the amino acid sequence NH₂-Gly-Val-Asn-Ala-Cys-Ser-Ser-Leu-Phe-COOH and a cyclic thioester bond between Cys and COOH.

17. The use of claim 14, the peptide having the amino acid sequence NH₂-Tyr-Ile-Asn-Cys-Asp-Phe-Leu-Leu-COOH and a cyclic thioester bond between Cys and COOH.

18. The use of claim 10, the medicament comprising more than one peptide which inhibits agr-rnaIII transcription in S. aureus.

19. A method of inhibiting agr-rnaIII transcription in S. aureus, the method comprising adding to the cells a peptide containing six to twelve amino acids in length and comprising amino acid number 28 from the AgrD region of a staphylococci bacterium, or an analogue thereof.

20. The method of claim 19, wherein the staphylococci bacterium is selected from the group consisting of S. aureus, S. capitis, S. caprae, S. carnosus, S. caseolyticus, S. epidermidis, S. haemolyticus, S. hominis, S. hyicus subsp. hyicus, S. hyicus subsp. chromo, S. kloosii, S. lentus, S. lugdunensis, S. sciuri, S. simulans and S. xylosus.

21. The method of claim 20, wherein the staphylococci bacterium is S. lugdunensis.

22. The method of claim 21, the peptide having the amino acid sequence NH₂-Asp-Ile-Cys-Asn-Ala-Tyr-Phe-COOH and a cyclic thioester bond between Cys and COOH.

23. The method of claim 20, wherein the staphylococci bacterium is S. aureus.

24. The method of claim 23, the peptide having the amino acid sequence NH₂-Tyr-Ser-Thr-Cys-Asp-Phe-Ile-Met-COOH and a cyclic thioester bond between Cys and COOH.

25. The method of claim 23, the peptide having the amino acid sequence NH₂-Gly-Val-Asn-Ala-Cys-Ser-Ser-Leu-Phe-COOH and a cyclic thioester bond between Cys and COOH.

26. The method of claim 23, the peptide having the amino acid sequence NH₂-Tyr-Ile-Asn-Cys-Asp-Phe-Leu-Leu-COOH and a cyclic thioester bond between Cys and COOH.

27. The method of claim 19, comprising more than one peptide which inhibits agr-rnaIII transcription in S. aureus.

## Patentansprüche

1. Arzneimittel, das ein gereinigtes Peptid, das die *agr*-*rnaIII*-Transkription in *S. aureus* inhibiert und sechs bis zwölf Aminosäuren in der Länge und Aminosäure Nummer 28 aus der AgrD-Region eines Staphylokokkenbakteriums enthält, oder ein Analogon davon, das die *agr-rnaIII*-Transkription in *S*. *aureus* inhibiert, und einen pharmazeutisch verträglichen Träger umfasst.

2. Arzneimittel nach Anspruch 1, wobei das Staphylokokkenbakterium aus der Gruppe ausgewählt ist, die aus *S. aureus, S. capitis, S. caprae, S. carnosus, S. caseolyticus, S. epidermidis, S. haemolyticus, S. hominis, S. hyicus* Subsp. *hyicus, S. hyicus* Subsp. *chromo, S. kloosii, S. lentus, S. lugdunensis, S. sciuri, S. simulans* und *S. xylosus* besteht.

3. Arzneimittel nach Anspruch 2, wobei das Staphylokokkenbakterium *S. lugdunensis* ist.

4. Arzneimittel nach Anspruch 3, wobei das Peptid die Aminosäuresequenz NH₂-Asp-Ile-Cys-Asn-Ala-Tyr-Phe-COOH und eine cyclische Thioesterbindung zwischen Cys und COOH besitzt.

5. Arzneimittel nach Anspruch 2, wobei das Staphylokokkenbakterium *S. aureus* ist.

6. Arzneimittel nach Anspruch 5, wobei das Peptid die Aminosäuresequenz NH₂-Tyr-Ser-Thr-Cys-Asp-Phe-Ile-Met-COOH und eine cyclische Thioesterbindung zwischen Cys und COOH besitzt.

7. Arzneimittel nach Anspruch 5, wobei das Peptid die Aminosäuresequenz NH₂-Gly-Val-Asn-Ala-Cys-Ser-Ser-Leu-Phe-COOH und eine cyclische Thioesterbindung zwischen Cys und COOH besitzt.

8. Arzneimittel nach Anspruch 5, wobei das Peptid die Aminosäuresequenz NH₂-Tyr-Ile-Asn-Cys-Asp-Phe-Leu-Leu-COOH und eine cyclische Thioesterbindung zwischen Cys und COOH besitzt.

9. Arzneimittel nach Anspruch 1, das mehr als ein Peptid umfasst, das die *agr*-*rnaIII*-Transkription in *S. aureus* inhibiert.

10. Verwendung eines Peptids, das die *agr-rnaIII*-Transkription in *S. aureus* inhibiert, zur Herstellung eines Arzneimittels für die Behandlung oder Verhütung einer von *S. aureus* verursachten Infektion oder Erkrankung, wobei das Peptid sechs bis zwölf Aminosäuren in der Länge und Aminosäure Nummer 28 aus der AgrD-Region eines Staphylokokkenbakteriums enthält, oder eines Analogons davon, das die *agr-rnaIII*-Transkription in *S. aureus* inhibiert.

11. Verwendung nach Anspruch 10, wobei das Staphylokokkenbakterium aus der Gruppe ausgewählt ist, die aus *S. aureus, S. capitis, S. caprae, S. carnosus, S. caseolyticus, S. epidermidis, S. haemolyticus, S. hominis, S. hyicus Subsp. hyicus, S.* *hyicus Subsp. chromo, S. kloosii, S. lentus, S. Lugdunensis, S. sciuri, S. simulans* und *S. xylosus* besteht.

12. Verwendung nach Anspruch 11, wobei das Staphylokokkenbakterium *S. lugdunensis* ist.

13. Verwendung nach Anspruch 12, wobei das Peptid die Aminosäuresequenz NH₂-Asp-Ile-Cys-Asn-Ala-Tyr-Phe-COOH und eine cyclische Thioesterbindung zwischen Cys und COOH besitzt.

14. Verwendung nach Anspruch 11, wobei das Staphylokokkenbakterium *S*. *aureus* ist.

15. Verwendung nach Anspruch 14, wobei das Peptid die Aminosäuresequenz NH₂-Tyr-Ser-Thr-Cys-Asp-Phe-Ile-Met-COOH und eine cyclische Thioesterbindung zwischen Cys und COOH besitzt.

16. Verwendung nach Anspruch 14, wobei das Peptid die Aminosäuresequenz NH₂-Gly-Val-Asn-Ala-Cys-Ser-Ser-Leu-Phe-COOH und eine cyclische Thioesterbindung zwischen Cys und COOH besitzt.

17. Verwendung nach Anspruch 14, wobei das Peptid die Aminosäuresequenz NH₂-Tyr-Ile-Asn-Cys-Asp-Phe-Leu-Leu-COOH und eine cyclische Thioesterbindung zwischen Cys und COOH besitzt.

18. Verwendung nach Anspruch 10, wobei das Arzneimittel mehr als ein Peptid umfasst, das die *agr-rnaIII*-Transkription in *S. aureus* inhibiert.

19. Verfahren zum Inhibieren der *agr-rnaIII*-Transkription in *S. aureus,* das die Zugabe eines Peptids, das sechs bis zwölf Aminosäuren in der Länge und Aminosäure Nummer 28 aus der agrD-Region eines Staphylokokkenbakteriums enthält, oder eines Analogons zu den Zellen umfasst.

20. Verfahren nach Anspruch 19, wobei das Staphylokokkenbakterium ist aus der Gruppe ausgewählt, die aus *S*. *aureus, S. capitis, S. caprae, S. carnosus, S. caseolyticus, S. epidermidis, S. haemolyticus, S. hominis, S. hyicus Subsp. hyicus, S. hyicus* Subsp. *chromo, S. kloosii, S. lentus, S. lugdunensis, S. sciuri, S. simulans* und *S*. *xylosus* besteht.

21. Verfahren nach Anspruch 20, wobei das Staphylokokkenbakterium *S. lugdunensis* ist.

22. Verfahren nach Anspruch 21, wobei das Peptid die Aminosäuresequenz NH₂-Asp-Ile-Cys-Asn-Ala-Tyr-Phe-COOH und eine cyclische Thioesterbindung zwischen Cys und COOH besitzt.

23. Verfahren nach Anspruch 20, wobei das Staphylokokkenbakterium *S. aureus* ist.

24. Verfahren nach Anspruch nach 23, wobei das Peptid die Aminosäuresequenz NH₂-Tyr-Ser-Thr-Cys-Asp-Phe-Ile-Met-COOH und eine cyclische Thioesterbindung zwischen Cys und COOH besitzt.

25. Verfahren nach Anspruch 23, wobei das Peptid die Aminosäuresequenz NH₂-Gly-Val-Asn-Ala-Cys-Ser-Ser-Leu-Phe-COOH und eine cyclische Thioesterbindung zwischen Cys und COOH besitzt.

26. Verfahren nach Anspruch 23, wobei das Peptid die Aminosäuresequenz NH₂-Tyr-Ile-Asn-Cys-Asp-Phe-Leu-Leu-COOH und eine cyclische Thioesterbindung zwischen Cys und COOH besitzt.

27. Verfahren nach Anspruch 19, das mehr als ein Peptid umfasst, das die *agr-rnaIII*-Transkription in *S. aureus* inhibiert.

## Revendications

1. Médicament comprenant un peptide purifié, qui inhibe la transcription de agr-rnaIII dans *S. aureus,* le peptide contenant six à douze acides aminés et comprenant l'acide aminé numéro 28 de la région AgrD d'une bactérie staphylocoque, ou un analogue de celui-ci, qui inhibe la transcription de agr-rnaIII dans *S. aureus,* et un support pharmaceutiquement acceptable.

2. Médicament selon la revendication 1, dans lequel la bactérie staphylocoque est choisie parmi le groupe consistant en *S. aureus, S. capitis, S. caprae, S. carnosus, S. caseolyticus, S. epidermis, S. haemolyticus, S. hominis, S. hyicus,* sous-espèce *hyicus, S. hyicus,* sous-espèce chromo, *S. kloosii, S. lentus, S. lugdunensis, S. sciuri, S. simulans* et *S. xylosus.*

3. Médicament selon la revendication 2, dans lequel la bactérie staphylocoque est *S. lugdunensis.*

4. Médicament selon la revendication 3, le peptide ayant la séquence des acides aminés NH₂-Asp-Ile-Cys-Asn-Ala-Tyr-Phe-COOH et une liaison thioester cyclique entre Cys et COOH.

5. Médicament selon la revendication 2, dans lequel la bactérie staphylocoque est *S*. *aureus.*

6. Médicament selon la revendication 5, le peptide ayant la séquence des acides aminés NH₂-Tyr-Ser-Thr-Cys-Asp-Phe-Ile-Met-COOH et une liaison thioester cyclique entre Cys et COOH.

7. Médicament selon la revendication 5, le peptide ayant la séquence des acides aminés NH₂-Gly-Val-Asn-Ala-Cys-Ser-Ser-Leu-Phe-COOH et une liaison thioester cyclique entre Cys et COOH.

8. Médicament selon la revendication 5, le peptide ayant la séquence des acides aminés NH₂-Tyr-Ile-Asn-Cys-Asp-Phe-Leu-Leu-COOH et une liaison thioester cyclique entre Cys et COOH.

9. Médicament selon la revendication 1, comprenant plus d'un peptide, qui inhibe la transcription de agr-rnaIII dans *S. aureus.*

10. Utilisation d'un peptide, qui inhibe la transcription de agr-rnaIII dans *S. aureus,* pour la préparation d'un médicament pour le traitement ou la prévention d'une infection ou maladie provoquée par *S*. *aureus,* le peptide contenant six à douze acides aminés et comprenant l'acide aminé numéro 28 de la région AgrD d'une bactérie staphylocoque, ou un analogue de celui-ci, qui inhibe la transcription de agr-rnaIII dans *S. aureus.*

11. Utilisation selon la revendication 10, dans laquelle la bactérie staphylocoque est choisie parmi le groupe consistant en *S. aureus, S. capitis, S. caprae, S. carnosus, S. casealyticus, S. epidermis, S. haemolyticus, S. hominis, S. hyicus,* sous-espèce *hyicus, S. hyicus,* sous-espèce *chromo, S. kloosii, S. lentus, S. lugdunensis, S. sciuri, S. simulans* et S. *xylosus.*

12. Utilisation selon la revendication 11, dans laquelle la bactérie staphylocoque est *S. lugdunensis.*

13. Utilisation selon la revendication 12, le peptide ayant la séquence des acides aminés NH₂-Asp-Ile-Cys-Asn-Ala-Tyr-Phe-COOH et une liaison thioester cyclique entre Cys et COOH.

14. Utilisation selon la revendication 11, dans laquelle la bactérie staphylocoque est *S. aureus.*

15. Utilisation selon la revendication 14, le peptide ayant la séquence des acides aminés NH₂-Tyr-Ser-Thr-Cys-Asp-Phe-Ile-Met-COOH et une liaison thioester cyclique entre Cys et COOH.

16. Utilisation selon la revendication 14, le peptide ayant la séquence des acides aminés NH₂-Gly-Val-Asn-Ala-Cys-Ser-Ser-Leu-Phe-COOH et une liaison thioester cyclique entre Cys et COOH.

17. Utilisation selon la revendication 14, le peptide ayant la séquence des acides aminés NH₂-Tyr-Ile-Asn-Cys-Asp-Phe-Leu-Leu-COOH et une liaison thioester cyclique entre Cys et COOH.

18. Utilisation selon la revendication 10, comprenant plus d'un peptide, qui inhibe la transcription de agr-rnaIII dans *S. aureus.*

19. Procédé d'inhibition de la transcription de agr-rnaIII dans *S*. *aureus,* le procédé comprenant l'addition aux cellules, d'un peptide contenant six à douze acides aminés et comprenant l'acide aminé numéro 28 de la région AgrD d'une bactérie staphylocoque, ou un analogue de celui-ci.

20. Procédé selon la revendication 19, dans lequel la bactérie staphylocoque est choisie parmi le groupe consistant en *S. aureus, S.* capitis, *S. caprae, S. carnosus, S. caseolyticus, S. epidermis, S. haemolyticus, S. hominis, S. hyicus,* sous-espèce *hyicus, S. hyicus,* sous-espèce *chromo*, *S. kloosii, S. lentus*, *S. lugdunensis, S. sciuri, S. simulans* et *S. xylosus.*

21. Procédé selon la revendication 20, dans lequel la bactérie staphylocoque est *S. lugdunensis.*

22. Procédé selon la revendication 21, le peptide ayant la séquence des acides aminés NH₂-Asp-Ile-Cys-Asn-Ala-Tyr-Phe-COOH et une liaison thioester cyclique entre Cys et COOH.

23. Procédé selon la revendication 20, dans lequel la bactérie staphylocoque est *S*. *aureus.*

24. Procédé selon la revendication 23, le peptide ayant la séquence des acides aminés NH₂-Tyr-Ser-Thr-Cys-Asp-Phe-Ile-Met-COOH et une liaison thioester cyclique entre Cys et COOH.

25. Procédé selon la revendication 23, le peptide ayant la séquence des acides aminés NH₂-Gly-Val-Asn-Ala-Cys-Ser-Ser-Leu-Phe-COOH et une liaison thioester cyclique entre Cys et COOH.

26. Procédé selon la revendication 23, le peptide ayant la séquence des acides aminés NH₂-Tyr-Ile-Asn-Cys-Asp-Phe-Leu-Leu-COOH et une liaison thioester cyclique entre Cys et COOH.

27. Procédé selon la revendication 19, comprenant plus d'un peptide, qui inhibe la transcription de agr-rnaIII dans *S. aureus.*
